# EUROPEAN PATENT SPECIFICATION

(11) **EP 4 342 502 B1**
(45) Date of publication and mention of the grant of the patent: **29.04.2026**
(21) Application number: 22196506.4
(22) Date of filing: 20.09.2022
(51) Int. Cl.: A61M 1/00, F04B 39/00, F04B 43/02, F04B 43/12, F04B 53/16

(54) **A PORTABLE ENCLOSURE FOR A NEGATIVE PRESSURE WOUND THERAPY DEVICE**
TRAGBARES GEHÄUSE FÜR EINE UNTERDRUCKWUNDTHERAPIEVORRICHTUNG
ENCEINTE PORTABLE POUR DISPOSITIF DE TRAITEMENT DE PLAIES PAR PRESSION NÉGATIVE

(43) Date of publication of application: 27.03.2024
(73) Proprietor: Mölnlycke Health Care AB, 431 21 Mölndal (SE)
(72) Inventor: BOJAHNDER, Otto, 44454 STENUNGSUND (SE); LANÇON, Océane, 42372 SÄVE (SE); WIKKELSÖ, Lene, 41268 GÖTEBORG (SE); LIND, Karin, 41526 GÖTEBORG (SE); DALL'ORSO, Sofia, 41713 GÖTEBORG (SE)
(74) Representative: Kransell & Wennborg KB

(56) References cited:
- WO-A1-2020/005532
- WO-A2-2013/171585
- US-A1- 2013 110 058
- US-A1- 2014 343 518
- US-A1- 2019 365 966

## Description

### TECHNICAL FIELD

The present disclosure generally relates to a portable enclosure for a mobile negative pressure wound therapy (NPWT) device, where the enclosure is arranged to reduce unwanted noise from a pump comprised with the negative pressure pump device.

### BACKGROUND

Negative pressure wound therapy (NPWT) is a technique that promotes healing of e.g. surgical, acute, and chronic wounds by the application of a sub-atmospheric pressure to the wound, using a negative pressure pump. The NPWT technique also permits less outside disturbance of the wound as well as for transportation of excess fluids away from the wound site. Generally, the NPWT technique has until now mainly been applied to a patient while in a hospital environment. However, recent product development now allows the technique to be used by a patient in a home environment.

When an NPWT device is used in such a home environment, it may be possible that the NPWT device is not operated and monitored by professional users, as compared to when the NPWT device is used in the mentioned hospital environment. Thus, it is desirable to further simplify the operational use of the NPWT device, for minimizing any errors in use and handling.

Introducing the NPWT device in the home environment may in some situations have further implications on the patient and/or e.g. a partner of the patient. Specifically, when the negative pressure pump is operating, the pump may generate a noise that may be perceived as disturbing for the patient and/or the partner of the patient.

One example of an NPWT device trying to mitigate this problem is disclosed in US8257328, where a direct current (DC) motor-driven pump comprised within a housing of the NPWT device is operated to achieve the negative pressure. In US8257328, a vibration damping tape, e.g., visco-elastic damping tape, may be applied to an outer surface of the pump to reduce vibration and its associated noise. The pump may also be contained within a sub-housing arranged within the housing of the NPWT device, where the sub-housing may be hollow or formed entirely of open cell molded foam, e.g., used as a silencer to provide sound mitigation by reducing the sound energy of during operation of the pump.

The solution presented in US8257328, specifically by arranging the pump within the mentioned sub-housing, generally reduces the level of noise generated during operation of the NPWT device. However, to achieve a desirable noise mitigating effect it will be necessary to introduce a substantial amount of the foam within the housing of the NPWT device, in turn result in a bulky NPWT device. Accordingly, there appears to be desirable to provide for other measures in mitigating the generated noise, while still ensuring that the NPWT device is safe to operate within the mentioned home environment.

Further attention is drawn to US20190365966A1, presenting a medical suction pump that comprises a pump housing, a pump assembly arranged in the pump housing and serving to generate an under pressure, and a device for sound damping. The device for sound damping has at least two elastic bearings for elastically supporting the pump assembly relative to the pump housing, wherein the bearings are arranged spaced apart from each other.

### SUMMARY

In view of above-mentioned and other drawbacks of the prior art, it is an object of the present disclosure to provide improvements in relation to efficient and safe operation of a NPWT device operating to establish a negative pressure within the sealed space formed by a wound cover in relation to a wound site.

According to an aspect of the present disclosure, it is therefore provided a portable enclosure for a mobile negative pressure wound therapy (NPWT) device as defined by claim 1.

The present disclosure is based upon the realization that further measures are needed to ensure that the operation of the NPWT device does not disturb the patient and/or e.g. a person in close contact with the patient. This is of specific importance in a home environment, since even in comparison low-level noise has shown to impact negative on the person and the possible relation with persons in close contact with the patient, such as a partner, a friend, or a colleague.

This is line with the present disclosure solved by providing the patient with a portable enclosure for the NPWT device, where the intention is to use the portable enclosure when the extra sound mitigation is needed. Nighttime usage as well as in generally interacting with fellows are thus just examples of when such extra sound mitigation is advantageously applied. Obvious scenarios include meetings, visits to restaurants and the cinema, etc.

The combination of the forming the two housing sections from a combination of an outer shell material and an inner insulation material has surprisingly shown to greatly reduce the noise level of the NPWT device to such an extent that noise stemming from the negative pressure pump is not perceived to be disturbed, even in a normal situation where the negative pressure pump is operation in an irregular manner (meaning that the noise level will be fluctuating over time).

It is generally desirable, but in some embodiments not absolutely necessary, to form the inner insulation material to match a shape of the NPWT device. The NPWT device will in such an embodiment have a "snug fit" to the inner insulation material, similar to how a camera may be positioned in a specifically adapted camera case. The combined insulation material for the first and the second housing section may in such an embodiment be formed to have a hollow portion towards the center of the portable enclosure, where the hollow portion matches the shape of the NPWT device. Hereby also a very compact design of the enclosure is achieved, which enhance the portability of the enclosure. Further, a particular outer shell design can be used to house several different variants of insulations, adapted after different pump designs.

Furthermore, since the operation of the NPWT device is in the mentioned home environment and not supervised by a skilled operator (such as e.g. a nurse), it is necessary to ensure that e.g. a sound indicative of a state of the NPWT device is still allowed to propagate to an outside of the portable enclosure. This is line with the present disclosure solved by introducing a sound duct extending through the outer shell material and the inner insulation material. The sound duct will thus at one end be positioned towards the NPWT device, and at the other end facing out of the portable enclosure. The end positioned towards the NPWT device is preferably arranged in relation to an area at the NPWT device where a volume of the sound generated by the sound generating device is as large as possible. Such an area may for example be defined by a sound outlet provided in a surface of the NPWT device, where the sound outlet possibly could be arranged to coincide with a position of the sound generating device at a printed circuit board (PCB) of the NPWT device.

It may however as an alternative be possible to allow the sound generating device to use a housing of the NPWT device to form part of the sound generating device. In such an embodiment it may thus be desirable to arrange the sound duct to engage and/or contact with the housing of the NPWT device.

In some embodiments it may additionally be desirable to allow for visual information from the NPWT device to be propagated outside of the portable enclosure. To achieve such a functionality, it may also be possible to equip the portable enclosure with a lightguide extending through the outer shell material and the inner insulation material and arranged to allow light emitted by a light source comprised with the NPWT device to be visible at an outside of the portable enclosure. The light emitted by the light source of the NPWT device will accordingly be visible to the patient even though the NPWT device is arranged inside of the closed portable enclosure. A diameter of the light guide may in one embodiment be between 1 - 10 mm.

To further heighten the sound mitigation effect of using the portable enclosure it may be desirable to ensure that the first housing section and the second housing section are adapted to completely encloses the NPWT device. It is of course necessary to ensure that the conduit is allowed to pass through e.g. a wall of the first and/or the second housing section of the portable enclosure. Thus, the conduit should not be interpreted as forming part of the NPWT device. Rather, the conduit forms together with the NPWT device and a wound cover part of a wound treatment system as will be discussed below in relation to the detailed description.

To ensure that the conduit is allowed to pass through the wall of the first and/or the second housing section of the portable enclosure it may accordingly be desirable to arrange the opening for passage conduit as a slot within the wall of the first and/or second housing section of the portable enclosure. The opening within the wall may alternatively be formed as a slit or a notch. The slot/slit/notch is desirably selected to ensure that the conduit is not "squeezed". Typically, a squeezed conduit could reduce a suction power of the NPWT device.

It may in some embodiments be desirable to also introduce a slot within a wall of the other one of the housing sections. Such an embodiment may be implemented with a desire to ensure that the NPWT device is "correctly" positioned within the portable enclosure, ensuring that the NPWT device is tightly fitted within the portable enclosure and align with possible hollow portions of the respective first and second housing sections.

It may generally be desirable to select the outer shell material to be of plastic or paper. From a cost perspective it may be suitable to select a paper material, such as for example of cardboard. That said, making use of a plastic material may allow for a prolonged use of the enclosure, which thereby will reduce an overall cost of using the enclosure. A thickness of the outer shell material may generally be selected to ensure a sufficient robustness and sturdiness of the portable enclosure, and of course depending on the type of outer shell material. That said, to achieve the desired sound dampening effect it has shown to be desirable to select the outer shell material to have a thickness around 1 - 4 mm.

It may in some embodiments be desirable to form the first and the second housing section by using a molding or injection process. For paper it may generally be desirable to form the first and the second housing section using a paper press pulp machine. When the outer shell material is plastic, such as for example polypropylene and polyethylene, it may be possible to form the first and the second housing section using compression molding or injection molding.

The first and the second housing section may in some embodiments be provided as separate elements, for example connected together using a hinge. As an alternative, it may in a preferred embodiment be possible to form the first and the second housing section one single element. In such an embodiment, i.e. where the first and the second housing section is provided as a single element, it may be desirable to integrate the above mentioned hinge as a further portion, in the following defined as a hinge portion. The hinge portion may for example be provided as a living hinge. A living hinge is a small section of e.g. the single element that has an embedded hinge so the part can bend or fold. These are customarily used to create both a lid and a container as one single molded element, i.e. where for example the first housing section may be defined as the container and the second housing section may be defined as the lid. Living hinges are generally superior to other options because they can be opened and closed thousands of times without losing flexibility and strength.

As mentioned above, it is generally desirable to form the inner insulation material to match the shape of the NPWT device, thereby maximizing a volume of the inner insulation material provided within the portable enclosure when the portable enclosure is closed around the NPWT device. One type of suitable inner insulation material is a polymer material, such as for example having a cell structure. A possible suitable inner insulation material may as such be a foam material.

To achieve a desirable amount of noise damping it is in one embodiment preferred to select a thickness of the inner insulation material to be at least 5 mm, preferably at least 10 mm. The thickness of the inner insulation material may also as an alternative be selected depending on desired amount of attenuation of the noise from the negative pressure pump during its operation. In a preferred embodiment the attenuation of the noise from the negative pressure pump is at least 4 dBa, preferably at least 6 dBa.

As discussed above and defined in accordance with the present disclosure, the sound duct (as well as optionally the light guide) extending through the outer shell material and the inner insulation material of the second housing section. It may in some embodiments be desirable to reduce any sound damping effect of the inner insulation material by arranging the sound duct to comprise a duct material that is selected to be different from the inner insulation material. That is, if for example the inner insulation material is a foam material, the foam material may dampen sound waves propagating through the sound duct. Arranging a wall of the sound duct to have more dense material properties may greatly reduce such a dampening effect. However, the fact that the duct material that is selected to be different from the inner insulation material should be interpreted in the broadest sense, also including an embodiment where a material property of the inner insulation material has been altered. As an example of such an alteration, it could for example be possible to heat treat the inner insulation material at a surface towards the sound duct.

However, it may also be possible to line the sound duct with a material that has a lower sound dampening effect as compared to the inner insulation material, such as by providing an in comparison thin plastic film to the inner insulation material. As an alternative, the duct material may also or instead comprise a paper or plastic tube section. Using a paper tube section may have advantages from an environmental perspective. In a preferred embodiment a diameter of the sound duct is at least 2 mm, preferably at least 5 mm. The sound duct may have a circular cross section, but the cross section may of course be of any other shape (rectangular, triangular, etc.).

In one embodiment, each of the first and the second housing section are shaped to generally follow the shape of the NPWT device. Taking into account the desire to introduce around 10 mm of inner insulation material around all sides of the NPWT device will result in a portable enclosure having a height, a width and a length that is around 20 mm greater than the height, a width and a length of the NPWT device, i.e. a combination of the thickness of the outer shell material and the inner insulation material.

It may furthermore be desirable to equip the portable enclosure with a locking arrangement for selectively locking the first housing section to the second housing section. As such, the NPWT device may be safely positioned within the portable enclosure, ensuring that the NPWT device remains within the portable enclosure even when the patient is actively moving around. The locking arrangement may for example be selected from a group comprising a zipper, a buckle, and a hasp.

Since there is a desire to allow the patient to move around even when using the NPWT device, it is preferred to provide the portable enclosure with a carrying strap. The carrying strap may for example be provided as a "loop", allowing the portable enclosure to be carried around the neck of the patient. It is preferred to form integrated connection points for the carrying strap within the outer shell material, such as being part of e.g. one of the first and the second housing section. To ensure that the portable enclosure can be carried by the patient in the most suitable manner, it may in some embodiments be desirable to include at least one buckle for adapting a length of the carrying strap.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Fig. 1 conceptually illustrates a wound treatment system comprising an NPWT device arranged within a portable enclosure according to the present disclosure,
Fig. 2 provides a perspective view of portable enclosure inside which the NPWT device is positioned,
Figs. 3A and 3B show perspective and cross section views where a first and a second housing section of the portable enclosure have been joined together around the NPWT device, and
Fig. 4 presents a patient provided with the portable enclosure according to the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

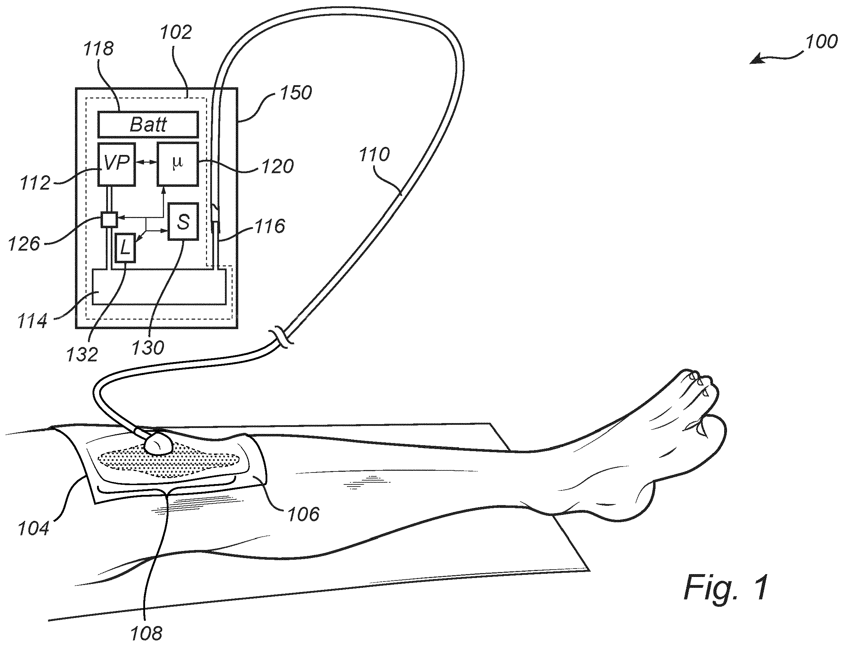

Turning now to the drawings and to Fig 1 in particular, there is conceptually illustrated a wound treatment system 100, comprising a NPWT device 102 in accordance with the present disclosure. The wound treatment system 100 further comprises a wound cover 104, the wound cover 104 being adapted to create a sealed space 106 defined in part by a wound surface 108, such as at the skin of a user/person, at or around a wound of the user/person. Additionally, the NPWT device 102 is fluidly connected to the wound cover 104 using e.g. a conduit 110. The conduit 110 may be of any suitable flexible conduit fabricated from elastomeric and/or polymeric materials. The NPWT device may alternatively comprise two conduits which are fluidly connected to the wound cover, and accordingly the opening in the enclosure may be designed to enclose both conduits.

The NPWT device 102 in turn comprises a negative pressure pump 112 adapted for establishing a negative pressure when the negative pressure pump 112 is operable, i.e. in an active state. The negative pressure pump 112 may be any type of pump that is biocompatible and maintains or draws adequate and therapeutic vacuum levels. Preferably, the negative pressure level to be achieved is in a range between about -20 mmHg and about -300 mmHg. In a possible embodiment of the present disclosure, a negative pressure range between about -80 mmHg and about -140 mmHg is used. In a possible embodiment of the present disclosure, the negative pressure pump 112 is either a diaphragm pump or a peristaltic pump, or the like, in which the moving parts draw the mentioned fluid from the wound cover 104.

The negative pressure pump 112 is fluidly connected to a canister 114, the canister 114 also forming part of the NPWT device 102. The canister 114 may be formed from e.g. molded plastic or the like, and possibly being a detachable component of the NPWT device 102. As mentioned above, the canister 114 is preferably at least partly transparent/translucent to permit viewing into the interior of the canister 114 to assist the user in determining the remaining capacity of the canister 114.

For ease of understanding of the following discussion of the present disclosure, it should be understood that the expressions "negative pressure", "sub-atmospheric pressure", "reduced pressure", as used interchangeably herein, generally refer to a pressure less than a local ambient pressure, such as the ambient pressure in a local environment external to a sealed therapeutic environment provided by a wound cover or dressing. In many cases, the local ambient pressure may also be the atmospheric pressure at which a patient is located. Unless otherwise indicated, values of pressure stated herein are gauge pressures. Similarly, references to increases in negative pressure typically refer to a decrease in absolute pressure, while decreases in negative pressure typically refer to an increase in absolute pressure.

An inlet port 116 is formed at the canister 114, for allowing connection to the conduit 110. The inlet port 116 may also be formed elsewhere at the NPWT device 102, however still fluidly connected to the canister 114. The connection between the inlet port 116 and the conduit 110 is a sealed connection, thus ensuring that no leakage is formed at the inlet port 116 during normal operation of the NPWT device 102. The conduit 110 is preferably releasably connected to the inlet port 116 through conventional means including a friction fit, bayonet coupling, snap fit, barbed connector, or the like. The inlet port 116 may be molded/formed from the same material and/or at the same time as forming the canister 114.

The NPWT device 102 further comprises a battery 118 for powering the NPWT device 102. The battery 118 may preferably be of the rechargeable type but may alternatively be arranged to be disposable and thus to be changed once discharged. A specifically adapted battery pack may be used in relation to some embodiment of the present disclosure.

The NPWT device 102 also comprises a control unit 120, electrically connected to the battery 118 and adapted to control an operation of the negative pressure pump 112. The control unit 120 may include a microprocessor, microcontroller, programmable digital signal processor or another programmable device. The control unit 120 may also, or instead, each include an application specific integrated circuit, a programmable gate array or programmable array logic, a programmable logic device, or a digital signal processor. Where the control unit 120 includes a programmable device such as the microprocessor, microcontroller or programmable digital signal processor mentioned above, the processor may further include computer executable code that controls operation of the programmable device.

In line with the present disclosure, the NPWT device 102 further comprises a control circuitry 122 provided externally from the control unit 120 and arranged to generally control the operation of the negative pressure pump 112, specifically for ensuring that the operation of the negative pressure pump 112 may be swiftly terminated in case the NPWT device 102 starts to operate outside of what is considered to be a normal behavior, as has been discussed above.

In addition, the NPWT device 102 comprises at least one pressure sensor 126 arranged in fluid connection with the negative pressure pump 112.

During use of the NPWT device 102, the wound cover 104 is arranged at a wound site of the user/patient, forming the sealed space 106. The conduit 110 is provided to fluidly connect the wound cover 104 to the inlet port 116 of the NPWT device 102. The NPWT device 102 is then activated, e.g. by the user/patient, for example by pressing a start button. When activated, the negative pressure pump 112 will start to evacuate air through the canister 114, the inlet port 116, the conduit 110 and the sealed space 106 formed by the wound cover 104. Accordingly, the negative pressure will be created within the sealed space 106. In case a liquid has been formed at the wound site, this liquid from the wound site may at least partly be "drawn" from the wound site, through the conduit 110, the inlet port 116 and into the canister 114. The amount of liquid (possibly defined as exudate) that is drawn from the wound and collected in the canister will depend on the type of wound that is being treated as well as the type of wound dressing used. For example, in case an absorbent dressing is used, the liquid may be absorbed and collected both in the canister and the wound dressing, whereas if a dressing with no or little absorption capacity is used most or all of the liquid from the wound site may be collected in the canister. A suitable filter member (not shown in Fig. 1) is arranged between the canister 114 and the negative pressure pump 112 to ensure that no fluid is allowed to pass to the negative pressure pump 112 from the canister 114.

In other embodiments (not shown), the NPWT device may be void of a canister, wherein the negative pressure pump is fluidly connected to an absorbent dressing which function to absorb all liquid drawn from the wound.

The NPWT device 102 may also, as indicated above comprise a speaker element 130 and thereto connected circuitry for driving the speaker element 130, where the speaker element 130 is connected to the control unit 120. The speaker element 130 is generally used for informing the patient of a specific state of the NPWT device 102, such as in case there is some form of problem relating to the operation of the NPWT device 102. Such an example may for example be an unwanted leakage in relation to the conduit 110 and/or the wound cover 104 or unwanted blockage of the conduit 110, where the unwanted leakage or blockage is identified by the control unit 120. The speaker element 130 will then be activated and adapted to play a specific and thereto related sound to inform the patient. Further possible dedicated informative sounds may relate to the canister 114 being full, the NPWT device 102 operating in an irregular manner, the battery 118 getting close to completely discharged, etc.

The NPWT device 102 may also comprise a light source 132 connected to the control unit 120. The light source 132 is typically, likewise as the speaker element 130, provided for communicating information to the patient. Such information may, as with the speaker element 130, include a specific state of the NPWT device 102, such as in case there is some form of problem relating to the operation of the NPWT device 102.

Furthermore, the wound treatment system 100 comprises the portable enclosure 150 according to the present disclosure, for reducing a level of noise generated during operation of the NPWT device 102, such as when the negative pressure pump 112 is activated.

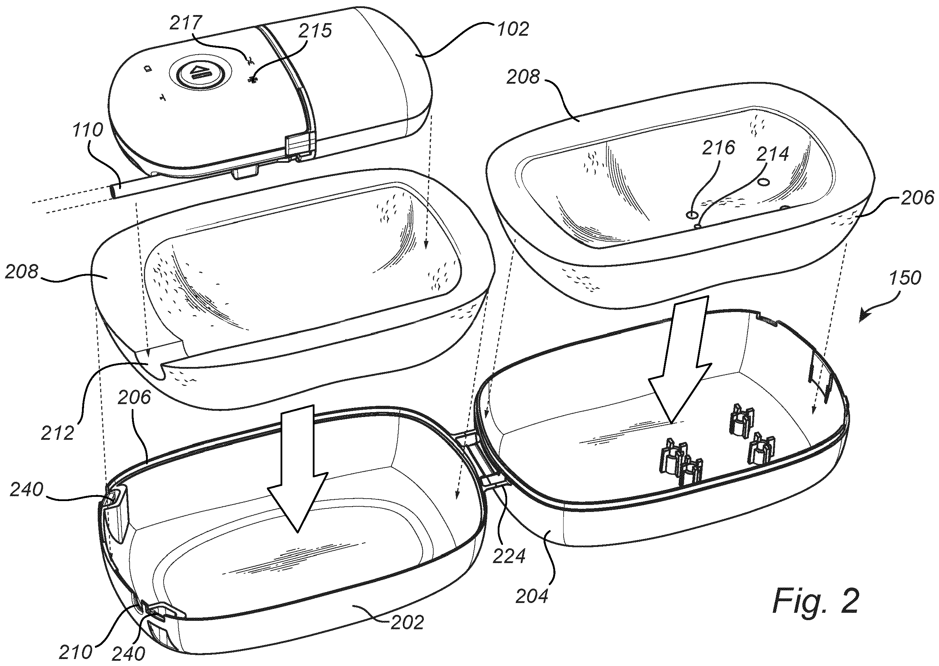

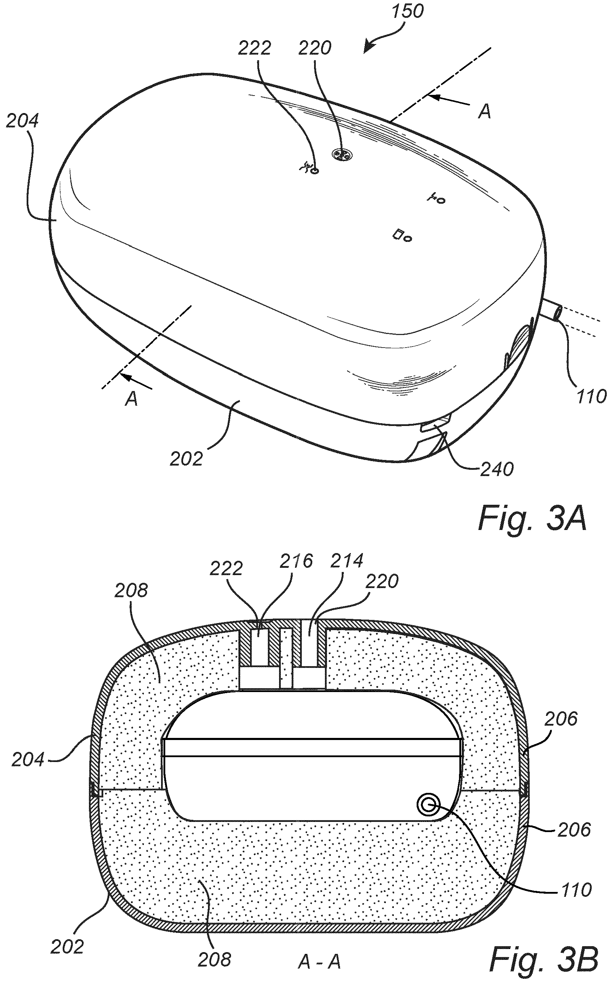

With further reference to Figs. 2, 3A and 3B, the portable enclosure 150 comprises a first housing section 202 and a second housing section 204. In the illustration as is shown in Figs. 2, 3A and 3B, the first housing section 202 is shown as a ("bottom") container portion of the portable enclosure 150 and the second housing section as a lid portion of the portable enclosure 150. The portable enclosure 150 is in Figs. 3A and 3B shown to have a shape that resembles a shape of the NPWT device 102, generally with "rounded corners" to ensure that no pain from a sharp corner is inflicted to the patient when the portable enclosure 150 is provided in close arrangement with the patient. It is of course possible to select any shape or form to achieve the desired effect according to the present disclosure.

The first housing section 202, the container portion of the portable enclosure 150, is formed from and comprises an outer shell material 206 made from plastic and an inner insulation material 208 made from a foam material. Other materials may of course be used with the context and scope of the present disclosure.

The inner insulation material 208 is in turn pre-cut or otherwise shaped (such as by molding) to correspond to a shape of the (bottom of the) NPWT device 102. As can be seen, the inner insulation material 208 is also pre-cut in relation to the conduit 110, and further provided with a slot 210 to allow the conduit 110 to pass through a wall of the first housing section 202.

The second housing section 204, the lid portion of the portable enclosure 150, is also formed from and comprises a corresponding outer shell material 206 made from plastic and a corresponding inner insulation material 208 made from a foam material. It is of course possible to use different materials for the lid portion as compared to the bottom portion.

The insulation material 208 of the second housing section 204 is also pre-cut or otherwise shaped to correspond to the shape of the (top of the) NPWT device 102. It may however, depending on the amount of inner insulation material 208 provided in relation to the first housing section 202 to allow the inner insulation material 208 to be essentially flat and to be slightly compressed when the first 202 and the second 204 housing sections are joined together.

In the illustration as shown in Figs. 2, 3A and 3B, the first housing section 202 and the second housing section 204 is provided as a single element, joined together using a living hinge 224 forming part if the single element and manufactured as has been discussed above. It may of course, and within the scope of the present disclosure, be possible to form the first housing section 202 and the second housing section 204 as separate elements, joined together with a separate hinge.

The second housing section 204 is further provided with an elongated sound duct 214 that extends from an inside of the second housing section 204, through the inner insulation material 208 and out through the outer shell material 206. One end of the elongated sound duct 214 is specifically positioned in such a manner that it aligns with a sound outlet position 220 at the second housing section 204 where sound from the speaker element 130 is "outputted" outside of the portable enclosure 150, as is specifically illustrated in Fig. 3. Outputting the sound from the speaker element 130 may be dependent on the implementation selected for the NPWT device 102. In one embodiment, and as illustrated in Fig. 2, the NPWT device 102 is provided with an opening 215 in a housing of the NPWT device 102, i.e. aligning with the other end of the sound duct 214.

It may however as an alternative be possible to arrange the speaker element 130 (in the form of an actuator) to be connected to the housing of the NPWT device 102, meaning that the housing of the NPWT device 102 will "vibrate" when the speaker element 130. In such an embodiment it could be possible to arrange the first end of the sound duct 214 to be arranged to coincide with a suitable position of the housing of the NPWT device 102. In Figs. 3A and 3B the sound duct 214 is presented in a preferred embodiment where the sound duct 214 is kept as "short as possible" and perpendicular to a flat outer surface of the second housing section 204, thereby maximining the output of sound generated by the speaker element 130 to an outside of the portable enclosure 150. It is however possible to arrange the sound duct 214 in any other way to achieve the general desired effect of the present disclosure.

Similarly, light emitted by the light source 132 may be propagated through light guide 216 at one end connected to a light outlet position 222 at the second housing section 204. The light guide 216 extends from an inside of the second housing section 204, through the inner insulation material 208 and out through the outer shell material 206. Accordingly, the other end of the light guide 216 is specifically positioned in such a manner that it aligns with a light outcoupling position 217 at the NPWT device 102, where light from the light source 132 is emitted to an outside of the NPWT device 102.

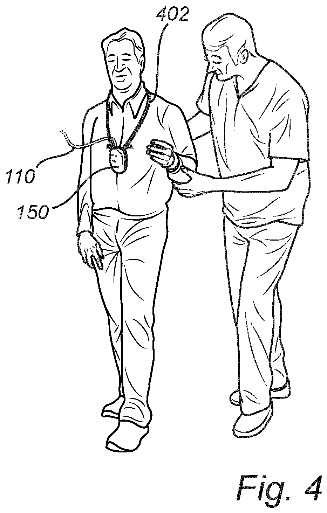

As is further illustrated in Figs. 2 and 3A, the first housing section 202 is provided with two integrated connection points 240 adapted for connecting a strap 402 (as shown in Fig. 4). Alternatively, the connection points 240 could be provided as separate elements in relation to the portable enclosure 150, however the cost for manufacturing of the portable enclosure 150 may generally be lower if the connection points 240 are provided as integrated with the first housing section 202.

The portable enclosure 150 is additionally provided with a locking arrangement 250 for selectively locking the first housing section 202 to the second housing section 204. In Fig. 3A the locking arrangement 250 is provided as a buckle but could likewise be arranged as a hasp. It could also be possible to lock the first housing section 202 to the second housing section 204 using a zipper.

Turning finally to Fig. 4, presenting a patient provided with the portable enclosure 150 according to the present disclosure. As can be understood from Fig. 4, the portable enclosure 150 her encloses the NPWT device 102, such that the conduit 110 extends out from the portable enclosure 150. The conduit 110 is intended to be connected to a wound cover (not shown). It may be possible to provide a "clip" (not shown) for directing conduit 110 to minimize a risk of folding the conduit 110. Folding the conduit 110 could possibly resulting in loss of suction power.

The patient is shown to carry the portable enclosure 150 around his neck, by means of strap 402 that has been looped around the neck of the patient and at each end are connected to the connection points 240 provided at the first housing section 202.

Although the figures may show a sequence, the order of the steps may differ from what is depicted. Also, two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps. Furthermore, in the claims, the word "comprising" does not exclude other elements or steps, and the indefinite article "a" or "an" does not exclude a plurality.

## Claims

1. A portable enclosure (150) for a mobile negative pressure wound therapy (NPWT) device (102), the NPWT device comprising a negative pressure pump (112) arranged within a housing of the NPWT device and connected to a wound cover using a conduit (110), wherein the enclosure is adapted to be removable and comprises:
- a first housing section (202),
- a second housing section (204), wherein the second housing section is arranged to join the first housing section to enclose the NPWT device, and
- a hinge arranged to join the first and the second housing section,
wherein:
- the first and the second housing sections each comprise an outer shell material (206) and an inner insulation material (208),
- at least one of the first or the second housing section is provided with an opening (210, 212) for passage of the conduit through a wall of the first or the second housing section, **characterized in that**:
- the second housing section is provided with a sound duct (214) extending through the outer shell material and the inner insulation material and arranged to allow a sound emitted by a sound generating device (130) comprised with the NPWT device to propagate to an outside of the portable enclosure.

2. The portable enclosure (150) according to claim 1, wherein the sound duct is positioned to coincide with a sound outlet (220) formed at the NPWT device.

3. The portable enclosure (150) according to any one of claims 1 and 2,
wherein the opening for passage of the conduit is provides as a slot (210) within a wall of the first housing section.

4. The portable enclosure (150) according to claim 3, further comprising a slot (212) within a wall of the second housing section, wherein the slots are arranged to align when the first housing section and the second housing section are joined together.

5. The portable enclosure (150) according to any one of the preceding claims, wherein the outer shell material is of paper or plastic.

6. The portable enclosure (150) according to any one of the preceding claims, wherein the inner insulation material is formed from a polymer material.

7. The portable enclosure (150) according to any one of the preceding claims, wherein the sound duct comprises a duct material and the duct material is different from the inner insulation material.

8. The portable enclosure (150) according to any one of the preceding claims, wherein a thickness of the inner insulation material is at least 5 mm, preferably at least 10 mm.

9. The portable enclosure (150) according to any one of the preceding claims, wherein the outer shell material and the inner insulation material is selected to attenuate noise from the negative pressure pump when operating with at least 4 dBa.

10. The portable enclosure (150) according to any one of the preceding claims, wherein the sound emitted by the sound generating device is indicative of a state of the NPWT device.

11. The portable enclosure (150) according to any one of the preceding claims, further comprising a locking arrangement (250) for selectively locking the first housing section to the second housing section.

12. The portable enclosure (150) according to claim 11, wherein the locking arrangement is selected from a group comprising a zipper, a buckle, and a hasp.

13. The portable enclosure (150) according to any one of the preceding claims, further comprising a carrying strap.

14. The portable enclosure (150) according to claim 13, further comprising at least one buckle for adapting a length of the carrying strap.

15. The portable enclosure (150) according to any one of the preceding claims, further comprising a lightguide extending through the outer shell material and the inner insulation material and arranged to allow light emitted by a light source comprised with the NPWT device to be visible at an outside of the portable enclosure.

## Patentansprüche

1. Tragbare Umhüllung (150) für eine mobile Unterdruckwundtherapievorrichtung (UWT-Vorrichtung) (102), wobei die UWT-Vorrichtung eine Unterdruckpumpe (112) umfasst, die in einem Gehäuse der UWT-Vorrichtung angeordnet und unter Verwendung einer Leitung (110) mit einer Wundabdeckung verbunden ist, wobei die Umhüllung so ausgelegt ist, dass sie abnehmbar ist und Folgendes umfasst:
- einen ersten Gehäuseabschnitt (202),
- einen zweiten Gehäuseabschnitt (204), wobei der zweite Gehäuseabschnitt so angeordnet ist, dass er mit dem ersten Gehäuseabschnitt verbunden wird und sie so die UWT-Vorrichtung umhüllen, und
- ein Scharnier, das so angeordnet ist, dass es den ersten und den zweiten Gehäuseabschnitt verbindet,
wobei:
- der erste und der zweite Gehäuseabschnitt jeweils ein Außenhüllenmaterial (206) und einen inneren Dämmstoff (208) umfasst,
- der erste und/oder zweite Gehäuseabschnitt mit einer Öffnung (210, 212) zum Durchführen der Leitung durch eine Wand des ersten oder des zweiten Gehäuseabschnitts versehen ist, **dadurch gekennzeichnet, dass**:
- der zweite Gehäuseabschnitt mit einem Schallkanal (214) versehen ist, der sich durch das Außenhüllenmaterial und den inneren Dämmstoff erstreckt und so angeordnet ist, dass sich Schall, der von einer Schallerzeugungsvorrichtung (130) ausgesendet wird, den die UWT-Vorrichtung umfasst, aus der tragbaren Umhüllung hinaus leiten lässt.

2. Tragbare Umhüllung (150) nach Anspruch 1, wobei der Schallkanal so platziert ist, dass er mit einer Schallöffnung (220) zusammenfällt, die an der UWT-Vorrichtung ausgebildet ist.

3. Tragbare Umhüllung (150) nach einem der Ansprüche 1 und 2, wobei die Öffnung zum Durchführen der Leitung als Schlitz (210) in einer Wand des ersten Gehäuseabschnitts vorgesehen ist.

4. Tragbare Umhüllung (150) nach Anspruch 3, ferner umfassend einen Schlitz (212) in einer Wand des zweiten Gehäuseabschnitts, wobei die Schlitze nacheinander ausgerichtet angeordnet sind, wenn der erste Gehäuseabschnitt und der zweite Gehäuseabschnitt miteinander verbunden sind.

5. Tragbare Umhüllung (150) nach einem der vorhergehenden Ansprüche, wobei das Außenhüllenmaterial Papier oder Kunststoff ist.

6. Tragbare Umhüllung (150) nach einem der vorhergehenden Ansprüche, wobei der innere Dämmstoff aus einem Polymerwerkstoff gebildet ist.

7. Tragbare Umhüllung (150) nach einem der vorhergehenden Ansprüche, wobei der Schallkanal ein Kanalmaterial umfasst und sich das Kanalmaterial vom inneren Dämmstoff unterscheidet.

8. Tragbare Umhüllung (150) nach einem der vorhergehenden Ansprüche, wobei eine Dicke des inneren Dämmstoffs mindestens 5 mm, vorzugsweise mindestens 10 mm beträgt.

9. Tragbare Umhüllung (150) nach einem der vorhergehenden Ansprüche, wobei das Außenhüllenmaterial und der innere Dämmstoff so ausgewählt sind, dass sie Schall von der Unterdruckpumpe dämpfen, wenn sie mit mindestens 4dBA arbeitet.

10. Tragbare Umhüllung (150) nach einem der vorhergehenden Ansprüche, wobei der Schall, der von der Schallerzeugungsvorrichtung ausgesendet wird, auf einen Zustand der UWT-Vorrichtung schließen lässt.

11. Tragbare Umhüllung (150) nach einem der vorhergehenden Ansprüche, ferner umfassend eine Verschlussanordnung (250) zum gezielten Verschließen des ersten Gehäuseabschnitts mit dem zweiten Gehäuseabschnitt.

12. Tragbare Umhüllung (150) nach Anspruch 11, wobei die Verschlussanordnung aus einer Gruppe ausgewählt ist, die einen Reißverschluss, eine Schnalle und eine Haspe umfasst.

13. Tragbare Umhüllung (150) nach einem der vorhergehenden Ansprüche, die ferner einen Tragegurt umfasst.

14. Tragbare Umhüllung (150) nach Anspruch 13, die ferner mindestens eine Schnalle zum Anpassen einer Länge des Tragegurts umfasst.

15. Tragbare Umhüllung (150) nach einem der vorhergehenden Ansprüche, die ferner einen Lichtleiter umfasst, der sich durch das Außenhüllenmaterial und den inneren Dämmstoff erstreckt und so angeordnet ist, dass Licht, das von einer Lichtquelle ausgesendet wird, die in der UWT-Vorrichtung enthalten ist, außerhalb der tragbaren Umhüllung sichtbar ist.

## Revendications

1. Enceinte portable (150) pour un dispositif de traitement de plaie par pression négative (NPWT) mobile (102), le dispositif NPWT comprenant une pompe à pression négative (112) agencée à l'intérieur d'un boîtier du dispositif NPWT et reliée à un recouvrement de plaie à l'aide d'un conduit (110), dans laquelle l'enceinte est adaptée pour être amovible et comprend :
- une première section de boîtier (202),
- une deuxième section de boîtier (204), dans laquelle la deuxième section de boîtier est agencée pour joindre la première section de boîtier afin d'enfermer le dispositif NPWT, et
- une charnière agencée pour joindre la première section de boîtier et la deuxième section de boîtier,
dans laquelle :
- la première section de boîtier et la deuxième section de boîtier comprennent chacune un matériau d'enveloppe extérieur (206) et un matériau d'isolation intérieur (208),
- au moins l'une de la première section de boîtier et la deuxième section de boîtier est pourvue d'une ouverture (210, 212) pour un passage du conduit à travers une paroi de la première section de boîtier ou de la deuxième section de boîtier, **caractérisée en ce que** :
- la deuxième section de boîtier est pourvue d'un canal de son (214) s'étendant à travers le matériau d'enveloppe extérieur et le matériau d'isolation intérieur et agencé pour permettre à un son émis par un dispositif de génération de son (130) compris dans le dispositif NPWT de se propager vers un extérieur de l'enceinte portable.

2. Enceinte portable (150) selon la revendication 1, dans laquelle le canal de son est positionné pour coïncider avec une sortie de son (220) formée au niveau du dispositif NPWT.

3. Enceinte portable (150) selon l'une quelconque des revendications 1 et 2, dans laquelle l'ouverture pour un passage du conduit est prévue sous forme de fente (210) à l'intérieur d'une paroi de la première section de boîtier.

4. Enceinte portable (150) selon la revendication 3, comprenant en outre une fente (212) à l'intérieur d'une paroi de la deuxième section de boîtier, dans laquelle les fentes sont agencées pour s'aligner lorsque la première section de boîtier et la deuxième section de boîtier sont jointes l'une à l'autre.

5. Enceinte portable (150) selon l'une quelconque des revendications précédentes, dans laquelle le matériau d'enveloppe extérieur est du papier ou du plastique.

6. Enceinte portable (150) selon l'une quelconque des revendications précédentes, dans laquelle le matériau d'isolation intérieur est un matériau polymère.

7. Enceinte portable (150) selon l'une quelconque des revendications précédentes, dans laquelle le canal de son comprend un matériau de canal et le matériau de canal est différent du matériau d'isolation intérieur.

8. Enceinte portable (150) selon l'une quelconque des revendications précédentes, dans laquelle une épaisseur du matériau d'isolation intérieur est d'au moins 5 mm, de préférence d'au moins 10 mm.

9. Enceinte portable (150) selon l'une quelconque des revendications précédentes, dans laquelle le matériau d'enveloppe extérieur et le matériau d'isolation intérieur sont choisis pour atténuer un bruit de la pompe à pression négative lorsqu'elle fonctionne à au moins 4 dBa.

10. Enceinte portable (150) selon l'une quelconque des revendications précédentes, dans laquelle le son émis par le dispositif de génération de son est indicatif d'un état du dispositif NPWT.

11. Enceinte portable (150) selon l'une quelconque des revendications précédentes, comprenant en outre un agencement de verrouillage (250) pour verrouiller sélectivement la première section de boîtier et la deuxième section de boîtier.

12. Enceinte portable (150) selon la revendication 11, dans laquelle l'agencement de verrouillage est choisi dans un groupe comprenant une fermeture éclair, une boucle et un moraillon.

13. Enceinte portable (150) selon l'une quelconque des revendications précédentes, comprenant en outre une bandoulière.

14. Enceinte portable (150) selon la revendication 13, comprenant en outre au moins une boucle pour adapter une longueur de la bandoulière.

15. Enceinte portable (150) selon l'une quelconque des revendications précédentes, comprenant en outre un guide de lumière s'étendant à travers le matériau d'enveloppe extérieur et le matériau d'isolation intérieur et agencé pour permettre à la lumière émise par une source de lumière comprise dans le dispositif NPWT d'être visible à un extérieur de l'enceinte portable.
